# DEMANDE DE BREVET EUROPEEN

(11) **EP 1 232 758 A1**
(43) Date de publication de la demande: **21.08.2002**
(21) Numéro de dépôt: 01420041.4
(22) Date de dépôt: 19.02.2001
(51) Int. Cl.: A61K 47/10, A61K 48/00

(54) **Polynucléotide formulé en vue d'un transfert intracellulaire amélioré**

(71) Demandeur: Aventis Pasteur, 69007 Lyon (FR); Aventis Pharma, 92160 Antony (FR)
(72) Inventeur: Pitard, Bruno, 44300 Nantes (FR)
(74) Mandataire: Ayroles, Marie-Pauline

(57) **Abrégé**

L'invention a pour objet une composition pharmaceutique comprenant un polynucléotide et au moins 2 % (poids / volume), de préférence 2 à 10 %, d'un copolymère non-ionique de formule (I) OH(CH2CH2O)a(CH(CH3)CH2O)b(CH2CH2O)cH dans laquelle a, b et c sont tels que la portion polyoxypropylène possède un poids moléculaire compris entre 1450 et 2050 et les portions polyoxyethylène constituent entre 75 et 85 % (poids : poids) du copolymère. La composition est de préférence exempte de lipide cationique ou de phosphate de sodium. Le copolymère est destiné à l'amélioration du transfert ou de l'expression du polynucléotide dans ces cellules eucaryotes. Un exemple de copolymère répondant à la formule (I) est de manière typique le F68. Une composition selon l'invention est notamment utile dans les domaines de la thérapie génique, de la vaccination et de l'immunothérapie.

## Description

L'invention a pour objet une méthode pour transférer un polynucléotide dans des cellules eucaryotes et son application à divers champs thérapeutiques, y compris la vaccination.

Jusqu'à la fin des années 90, on s'accordait à penser que des transferts d'information génétique *in vivo* ne pouvaient avoir lieu que si le matériel génétique était soit encapsulé dans des liposomes, soit intégré dans un vecteur viral. Puis on a montré que de l'ADN en solution aqueuse isotonique une fois injecté par voie intramusculaire ou intradermique à l'aide d'une simple seringue pouvait exprimer chez le receveur l'information génétique pour laquelle il codait (Wolff et al, Science (1990) 247 : 1465). Néanmoins, il est apparu très vite que l'application directe de cette découverte à la thérapie génique ou à la vaccination n'était pas en pratique très aisée notamment dans la mesure où les quantités d'ADN requises étaient très importantes. On a donc alors songé à formuler le matériel génétique afin d'augmenter l'efficacité de transfection à l'intérieur des cellules et/ou dans le noyau.

Au cours de ces dix dernières années toute une gamme de produits ont été testés à cette fin. Il serait fastidieux de les nommer tous mais les plus connus d'entre eux sont notamment les microparticules de PLGA, les lipides cationiques tels que le DOTMA (chlorure de N-[1-2,2-(dioleyloxy)propyl]-N,N,N-triméthylammonium), les lipopolyamines et les polymères cationiques de type polylysine. Ces composés se complexent aux polynucléotides et sont sensés favoriser leur transfection. Néanmoins aucun résultat réellement satisfaisant n'a été enregistré ; les performances des polynucléotides formulés étant tout au plus équivalentes à celles des polynucléotides non-formulés.

On a par ailleurs constaté que certains de ces complexes pouvaient manquer de stabilité et une piste d'amélioration a consisté dans l'emploi d'un deuxième produit, tel qu'un agent de surface non-ionique venant stabiliser des complexes agents de transfection cationiques /polynucléotides (WO98/34648). Cet agent de surface peut notamment être un polooxyalkylène tel que le Pluronics™ F68 commercialisé par BASF, aussi connu sous le nom de Lutrol.

On a maintenant constaté qu'un composé tel que le F68 pouvait tout aussi bien agir directement sur le niveau de transfection et cela bien sûr en l'absence de toute molécule cationique, à condition qu'il soit ajouté en quantité suffisante pour que l'on puisse observer un taux de transfection accru par rapport à du matériel génétique non-formulé.

C'est pourquoi l'invention a pour objet une composition pharmaceutique comprenant un polynucléotide et au moins 2 % (poids / volume) d'un copolymère non-ionique de formule (I) OH(CH2CH2O)a(CH(CH3)CH2O)b(CH2CH2O)cH dans laquelle a, b and c sont tels que la portion polyoxypropylène possède un poids moléculaire compris entre 1450 and 2050 et les portions polyoxyethylène constituent entre 75 and 85 % (poids : poids) du copolymère.

Sous un autre aspect, l'invention a également pour objet l'usage d'un copolymère non-ionique de formule (I) OH(CH2CH2O)a(CH(CH3)CH2O)b(CH2CH2O)cH dans laquelle a, b et c sont tels que la portion polyoxypropylène possède un poids moléculaire compris entre 1450 et 2050 et les portions polyoxyethylène constituent entre 75 et 85 % (poids : poids) du copolymère dans la fabrication d'un médicament contenant un polynucléotide à titre de principe actif; le copolymère étant ajouté au polynucléotide à la concentration d'au moins 2 % (poids : volume) pour améliorer le transfert et/ou de l'expression du polynucléotide à l'intérieur des cellules de l'individu ayant besoin dudit médicament.

L'invention concerne également un procédé pour transférer un polynucléotide dans des cellules eucaryotes selon lequel on met en présence les cellules et le polynucléotide ; le polynucléotide étant formulé avec au moins 2 % (poids : volume) d'un copolymère non-ionique de formule (I) OH(CH2CH2O)a(CH(CH3)CH2O)b(CH2CH2O)cH dans laquelle a, b et c sont tels que la portion polyoxypropylène possède un poids moléculaire compris entre 1450 et 2050 et les portions polyoxyethylène constituent entre 75 et 85 % (poids : poids) du copolymère. De manière typique, il peut s'agir (i) d'une méthode de transfert de polynucléotide *in vivo* selon laquelle on administre une composition selon l'invention à un mammifère ou tout autre animal ; et (ii) une méthode de transfert de polynucléotide *in vitro* ou *ex vivo* selon laquelle on met en contact des cellules de mammifères (ou issues de tout autre animal) avec une composition selon l'invention. Dans le cas d'une méthode de transfert *ex vivo,* les cellules de mammifères ont été au préalable prélevées à partir d'un organisme et ont vertu à y être réimplantées après avoir incorporé le polynucléotide.

De manière avantageuse, une composition selon l'invention est exempte de lipide cationique et de phosphate de sodium. De manière optionnelle, une composition selon l'invention peut comprendre en outre un composé sélectionné dans le groupe constitué par le chlorure de sodium, le chlorure de potassium et le chlorure de magnésium ; de préférence en quantité isotonique ou hypertonique.

Pour usage aux fins de la présente invention, un copolymère non-ionique répondant à la description donnée ci-avant est de manière typique le Pluronic™ F68 commercialisé par BASF. Son poids moléculaire moyen est estimé à 8,400 et les parties hydrophiles de polyoxyethylène représentent environ 80 % du poids total. Ce produit se présente sous forme solide à 20°C. En solution aqueuse et à température ambiante, un copolymère de formule (I) forment des micelles sphériques dans une gamme assez large de concentration (par exemple entre 1 et 15 %). A température ambiante, une solution aqueuse contenant *e.g.* 1 à 15 % d'un copolymère de formule (I) se présente sous forme de liquide. A des températures plus élevées, la solution évolue vers une forme de gel, puis de pâte. Pour plus de renseignements, on peut consulter Alexandridis P., Current Opinion in Colloid & Interface Science (1997) 2 : 478 que l'on incorpore par référence.

De manière avantageuse, une composition pharmaceutique selon l'invention comprend de 2 à 15 % ; de préférence de 2 à 10 % ; de manière tout à fait préférée, environ 5 % d'un copolymère de formule (I).

Aux fins de la présente invention, le polynucléotide peut être aussi bien un polydesoxyribonucléotide qu'un polyribonucléotide. Leur origine importe peu : naturelle ou artificielle ; ADN génomique ou complémentaire ; ARN de transfert ou ribosomaux. Ils peuvent notamment être d'origine animale, humaine, végétale, bactérienne, virale, etc.

Leur fonction à titre de principe thérapeutique peut notamment consister à agir en tant que molécule anti-sens en venant contrôler l'expression des gènes ou la transcription d'ARNm. dans la cellule-hôte. Il peut aussi s'agir de polynucléotide capable de gouverner l'expression dans une cellule eucaryote d'un produit protéique d'intérêt.

Selon un mode particulier, le polynucléotide code pour une protéine, un polypeptide ou un peptide ayant un intérêt thérapeutique dont l'expression par la cellule-hôte permet de pallier un disfonctionnement de l'organisme récepteur. Une composition selon l'invention trouve donc un emploi en thérapie génique *in vivo* ou *ex vivo*.

Le polynucléotide peut aussi coder pour un produit protéique capable de générer une réponse immune à son encontre chez l'homme ou l'animal. Selon ce mode particulier de mise en oeuvre, l'invention conduit donc à la réalisation de vaccins ou de traitements immunothérapeutiques appliqués à l'homme ou à l'animal, notamment pour traiter ou prévenir des infections, *e.g.* virales ou bactériennes, ou des états cancéreux.

Pour usage dans les deux derniers modes de mise en oeuvre cités, le polynucléotide est avantageusement de l'ADN et se présente de préférence sous forme de vecteur *i.a*. de vecteur plasmidique. Un tel vecteur doit être non-infectieux et ne doit pas se répliquer chez l'organisme-hôte. Enfin, il doit être substantiellement dépourvu de capacité d'intégration dans le génome de l'organisme hôte. La séquence d'ADN codant pour le polypeptide thérapeutique ou antigénique doit être placée sous le contrôle d'éléments nécessaires à son expression dans l'organisme-hôte. A cette fin, il est très courant d'employer le promoteur précoce du Cytomégalovirus (CMV).

Comme vu précédemment, une composition pharmaceutique selon l'invention peut être utilisée à des fins de thérapie génique *in vivo* ou *ex vivo*. C'est pourquoi, selon un autre aspect, l'invention a aussi pour objet une méthode de traitement d'une maladie résultant de l'absence ou de la déficience d'un gène, selon laquelle (i) on administre à un patient ayant besoin d'un tel traitement une composition comprenant un polynucléotide incluant le gène apte à corriger la maladie et au moins 2 % (poids / volume) d'un copolymère non-ionique de formule (I) ; ou (ii) on prélève des cellules malades à partir d'un patient ayant besoin d'un tel traitement, on met ces cellules en contact avec une composition comprenant un polynucléotide incluant le gène apte à corriger la maladie et au moins 2 % (poids / volume) d'un copolymère non-ionique de formule (I) et on réimplante les cellules ainsi transfectées chez le patient.

Une composition utile dans le domaine de la thérapie génique emploie un polynucléotide comportant un gène thérapeutique c'est-à-dire un gène codant pour un produit protéique ayant un effet thérapeutique. Ce produit protéique peut être homologue vis-à-vis de la cellule cible (c'est-à-dire un produit qui est normalement exprimé dans la cellule cible lorsque celle-ci ne présente aucune pathologie). Dans ce cas là, l'expression de ce dernier consécutive à l'administration d'une composition selon l'invention, permet de pallier par exemple une expression insuffisante ou l'expression d'une protéine inactive ou faiblement active. Le gène thérapeutique peut aussi coder pour un mutant d'une protéine cellulaire ayant une stabilité accrue, une activité modifiée, etc. Le produit protéique peut être également hétérologue vis-à-vis de la cellule cible et par exemple compléter, apporter ou modifier une activité déficiente ou aberrante.

Dans des traitements thérapeutiques *in vivo,* une composition selon l'invention peut être administrée par la voie la plus appropriée au traitement sans exclusion particulière et d'une manière générale, il peut s'agir de la voie topique, cutanée, orale, rectale, vaginale, parentérale, intra nasale, intramusculaire, sous-cutanée, intraoculaire, intradermique, etc.

Une composition selon l'invention peut être aussi utile dans le domaine de la vaccination et de l'immunothérapie. Dans ce cas-là, elle emploie un polynucléotide comportant une séquence codant pour un produit antigénique de nature protéique (protéine, polypeptide, peptide ...) qui peut être par exemple un polypeptide exprimé dans les conditions naturelles par un agent infectieux quelconque (*e.g*. bactérie ou virus pathogène) ou un polypeptide de mammifères dont l'expression aberrante est caractéristique d'un état cancéreux (antigène associé à la tumeur). On parle alors de polypeptide spécifique d'un agent infectieux ou d'un état cancéreux.

L'application d'une composition selon l'invention au domaine de la vaccination est particulièrement intéressant dans la mesure où l'on a constaté que le copolymère non-ionique de formule (I) permet d'augmenter significativement la réponse anticorps vis-à-vis de l'antigène par comparaison avec ce qui est observé avec un polynucléotide non-formulé. On sait que le faible taux d'anticorps induits constitue l'une des limitations de la vaccination ADN ; ce mode de vaccination induisant en général de manière préférentielle une réponse immune de type cellulaire.

La propriété qui consiste à favoriser l'induction d'une réponse anticorps à un niveau convenable n'est pas propre au copolymère de formule (I). Des copolymères non-ioniques de type varié (polyols non-ioniques ou dérivés) permettent également de produire le même effet. A cette fin, il peut notamment s'agir de polyoxyalkylène avec des groupements alkylènes de longueur ou de conformation différente ou non au sein du polymère; en particulier de blocks copolymères de polyoxyethylène / polyoxypropylène tels que ceux décrits dans Paschalis P. (supra) *e.g.,* poloxamères et poloxamines, et notamment ceux répondant à la formule (II) OH(CH2CH2O)a(CH(CH3)CH2O)b(CH2CH2O)cH dans laquelle a, b et c sont tels que la portion polyoxypropylène possède un poids moléculaire compris entre 1000 et 4000 et les portions polyoxyethylène constituent entre 10 et 85 % (poids : poids) du copolymère.

C'est pourquoi l'invention a aussi pour objet l'usage d'un polynucléotide codant pour un produit protéique spécifique d'un agent infectieux ou d'un état cancéreux et d'un copolymère non-ionique, notamment ceux de formule (I) ou (II), dans la fabrication d'un médicament destiné au traitement ou à la prévention d'une maladie infectieuse ou d'un état cancéreux. Ce médicament ou composition vaccinale est en particulier indiqué pour induire une réponse immune notamment de type humoral visant au traitement ou à la prévention d'une maladie infectieuse ou d'un état cancéreux.

Une composition selon l'invention utile dans le domaine de la vaccination et de l'immunothérapie peut être administrée par toute voie en usage dans les domaines pré-cités ; notamment par voie muqueuse *e.g.* orale, intragastrique et intranasale, ou parentérale, *e.g.* intramusculaire, intradermique intra-épidermique et sous-cutanée. De manière avantageuse, une composition utile au traitement d'un état cancéreux est administrée au plus proche du site ou du tissu tumoral. Lorsque la composition vise au traitement d'une tumeur solide, l'administration peut s'effectuer au site même de la tumeur, notamment par injection directe..

D'une manière générale, la quantité de polynucléotide destinée à être administrée dépend de très nombreux facteurs tels que la maladie à traiter ou à prévenir, la nature même du polynucléotide *e.g.,* ADN / ARN antisens ou ADN plasmidique, la force du promoteur du vecteur plasmidique, l'activité biologique du produit exprimé par le gène, de la condition physique de l'individu ou de l'animal *i.a*. du mammifère auquel est destinée la composition (poids, âge, etc.), du mode d'administration et du type de formulation. En général, une dose efficace d'un point de vue thérapeutique ou prophylactique d'environ 10 µg à environ 5 mg, de préférence d'environ 100 µg à environ 5 mg ; de manière tout particulièrement préférée d'environ 250 µg à environ 3 mg peut être administrée à des humains adultes. L'administration peut être effectuée en dose unique ou répétée par intervalle.

Une composition selon l'invention peut être fabriquée de manière conventionnelle selon les règles en usage dans le domaine de la thérapie génique, des vaccins ou de l'immunothérapie. En particulier, une composition contient un véhicule acceptable d'un point de vue pharmaceutique et peut être sous forme solide *e.g.* lyophilisée, ou liquide. Si nécessaire, la forme solide peut être reconstituée en milieu liquide pour administration.

L'invention est illustrée ci-après par référence aux figures suivantes.

La Figure 1 présente le niveau d'expression de la phosphatase alcaline sécrétée (SeAP) chez des souris ayant reçu une injection par voie intra musculaire du plasmide VR-SeAP formulé ou non en présence de F68 à 5 %.

La Figure 2 présente l'évaluation par ELISA de la réponse anticorps anti-hémaglutinine chez des souris ayant reçu deux injections par voie intra musculaire de diverses doses du plasmide VR-HA formulé ou non en présence de F68 à 5 %.

La Figure 3 présente l'évaluation par ELISPOT de la réponse immune de type cellulaire induite chez des souris ayant reçu deux injections par voie intra musculaire de diverses doses du plasmide VR-HA formulé ou non en présence de F68 à 5 %.

### Exemple 1 : Transfection in vivo en présence de F68, du gène codant pour la phosphatase alcaline sécrétée.

Dans l'expérience rapportée ci-après, on étudie simplement le niveau d'expression du gène codant pour la phosphatase alcaline sécrétée (SeAP), après transfection *in vivo* en présence ou absence de F68 à 5 %. Pour ce faire, on utilise le plasmide VR-SeAP codant pour la phosphastase alcaline sécrétée. Ce plasmide a été construit à partir du plasmide VR1012 décrit dans Hartikka et al, Hum. Gene Ther. (1996) 7 (10) 1205. Il contient la séquence codant pour la SeAP placée sous le contrôle du promoteur IE1 du cytomégalovirus et du signal polyA du gène de l'hormone de croissance bovine.

Pour la préparation des échantillons entrant dans la mise en oeuvre du test on utilise une solution d'ADN à 200 µg / ml en NaCl 1,8 % 50 mM tampon Hepes (ADN 2X) que l'on mélange volume à volume avec une solution de F68 2X dans de l'eau. Un échantillon de contrôle est aussi réalisé (ADN nu en 0,9 % NaCl et 25 mM Hepes).

On constitue deux groupes de 6 souris femelles Balb/C âgées de 8 à 10 semaines. Les souris sont anesthésiées. Puis on leur injecte par voie intramusculaire 50 µl d'un échantillon dans chacun des *Tibialis* antérieurs. Les groupes sont les suivants :

| Groupe | µg ADN Dose / souris | Volume (µl) Dose / souris | Formulation |
|---|---|---|---|
| 1 | 10 | 100 | ADN nu |
| 2 | 10 | 100 | 5 % F68 |

Sept jours après l'injection, on effectue des prélèvements sanguins et à partir des séra on mesure l'expression du gène transfecté comme suit.

Le dosage de la SeAP est mis en oeuvre à l'aide du kit Clontech Ref. Great Escape K-2041-1. Brièvement, on distribue 15 µl de sérum par puits dans une plaque de 96 et on rajoute 45 µl de tampon de dilution 1X On laisse incuber 30 min à 65°C afin d'inactiver la phosphatase alcaline endogène puis on refroidit à 4°C et on laisse revenir à température ambiante. On ajoute alors 60 µl par puits de tampon test et on laisse incuber 5 min à température ambiante. Le contenu des puits est transféré dans une plaque noire de 96 puits (Microfluor, Dynatech). On ajoute alors 60 µl de CSPD (disodium 3-(4-methoxy-spiro(1,2-dioxetane-3,2'-(5'-chloro)tricyclo[3,3.1.1^{3.7}]decan)-4-yl)phenyl phosphate) dilué au 1/20 et on incube 20 min à température ambiante. Le signal de chemioluminescence témoignant de l'activité phosphatase alcaline est mesuré au luminomètre Victor-1420 (Wallac).

Une gamme étalon (dilutions de 10⁻⁷ à 10⁻¹ soit de 0.01 à 10000 pg/µl) de la SeAP est réalisée en utilisant la SeAP recombinante à 0.1 mg/ml fournie dans le kit. La droite de régression de cette gamme étalon permet de traduire l'expression de la SeAP en pg/ml, à partir des unités de lumière (RLU).

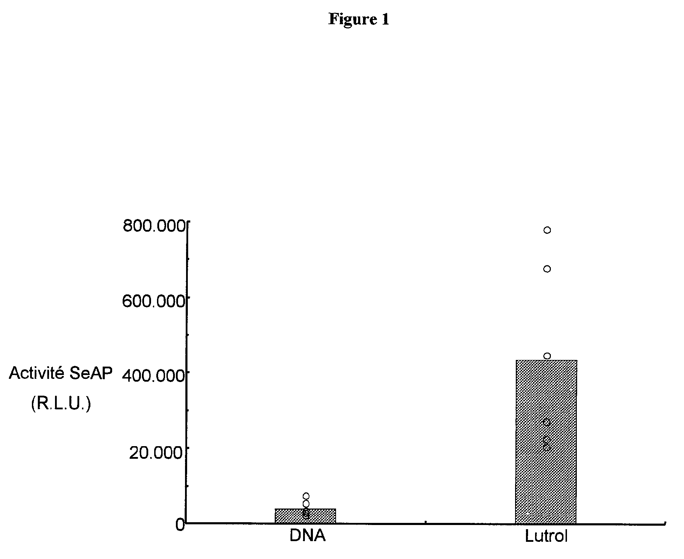

Les résultats sont présentés à la Figure 1 et indiquent que la présence de F68 5 % améliore d'environ 11 fois le niveau d'expression de la SeAP.

### Exemple 2 : Etude de la réponse immune induite par différentes doses d'ADN codant pour l'hémaglutinine du virus de la grippe (HA) formulé ou non avec du F68 à 5 %.

Dans l'expérience rapportée ci-après, on étudie les réponses immunes de type humoral et cellulaire induites par différentes doses de plasmide VR-HA formulé ou non avec du F68 à 5 %. Pour ce faire, on utilise le plasmide VR-HA codant pour l'hémaglutinine du virus de la grippe. Ce plasmide a été construit à partir du plasmide VR1012 décrit dans Hartikka et al, Hum. Gene Ther. (1996) 7 (10) 1205. Il contient la séquence codant pour l'hémaglutinine du virus de la grippe (A/PR8/34,H1N1) placé sous le contrôle du promoteur IE1 du cytomégalovirus et du signal polyA du gène de l'hormone de croissance bovine.

On constitue six groupes de 6 souris femelles Balb/C âgées de 8 à 10 semaines. Les souris sont anesthésiées. Puis on leur injecte par voie intramusculaire 50 µl d'un échantillon dans chacun des *Tibialis* antérieurs. On réalise deux séries d'injections identiques à J0 et J21. Les groupes sont les suivants :

| Groupes | VR-HA (µg) Dose / souris | Volume (µl) Dose / souris | Formulation |
|---|---|---|---|
| A | 1 | 100 | - |
| B | 10 | 100 | - |
| C | 50 | 100 | - |
| D | 1 | 100 | 5 % F68 |
| E | 10 | 100 | 5 % F68 |
| F | 50 | 100 | 5 % F68 |

Pour la préparation des échantillons entrant dans la mise en oeuvre du test on utilise une solution d'ADN 2X en NaCl 1,8 % 50 mM tampon Hepes (ADN 2X) que l'on mélange volume à volume avec une solution de F68 10 % dans l'eau.

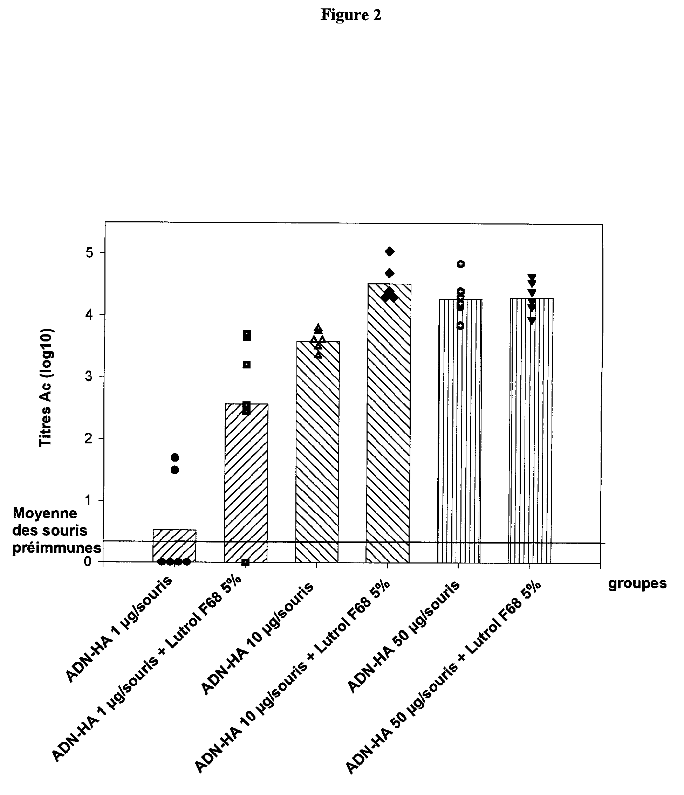

A J0, 14 et 35, on effectue des prélèvements sanguins, les sera sont recueillis et l'étude de la réponse humorale est mise en oeuvre par test ELISA tel que décrit dans Haensler et al, Vaccine (1999) 17 (7-8) : 628 en utilisant comme antigène de coating un virus de la grippe inactivé (A/PR/8/34). Les titres en anticorps sont calculés à partir d'une droite de régression à 4 paramètres d'un standard constitué par un sérum de souris anti-hémaglutinine A/PR/8/34 (Softmax software, Molecular devices, BioTimes). Le titre du standard a été au préalable déterminé à partir de 10 mesures indépendantes les unes des autres selon la formule OD(490 - 650 nm) x 10 / 1 /dilution. Un sérum est considéré comme positif si son titre spécifique est supérieur après immunisation d'au moins 0,5 log₁₀ par rapport à la moyenne du titre avant immunisation. Les résultats sont présentés dans la Figure 2.

On constate que le F68 à 5 % augmente notablement la réponse humorale induite par le plasmide VR-HA injecté à de faibles doses (1 et 10 µg). Quand la dose est importante (50 µg de plasmide) la réponse semble être au plafond et le F68 perd de son efficacité.

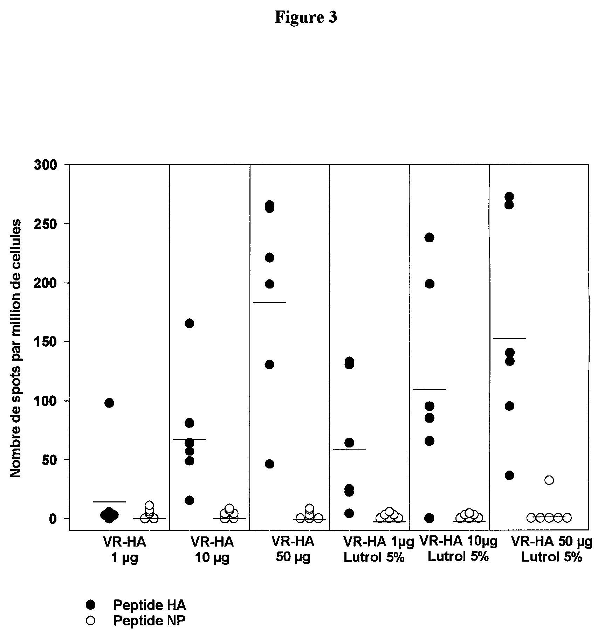

Les souris sont sacrifiées à J35 et les cellules de la rate sont récupérées pour mettre en oeuvre le test ELISPOT suivant : Des plaques de titrage en nitrocellulose (96 puits Multiscreen MA, Millipore) sont incubés avec un anticorps anti-IFN-gamma (Ref 18181, BD Pharmingen) à raison de 10 µg/ml en tampon phosphate (PBS) pendant une heure à température ambiante. Les plaques sont bloquées avec du milieu RPMI (Gibco BRL, Life technologies) pendant une heure puis lavées en PBS. Les cellules de la rate sont mise en suspension à raison de 2 x 10⁵ cellules/ml dans du milieu RPMI contenant 10 % de sérum de veau foetal, 20 U/ml d'IL-2 murine et des antibiotiques. Cent µl des suspensions cellulaires sont distribuées en trois exemplaires dans les plaques dont la préparation est décrite ci-avant. Le peptide correspondant à l'épitope CTL (lymphocyte T cytotoxique) de l'hémaglutinine A/PR/8/34 restreint par le complexe H-2K^{d} (IYSTVASSLVL) ou celui correspondant à l'épitope CTL de la nucléoprotéine du virus de la grippe A/PR/8/34 (TYQRTRALVTG, contrôle négatif) sont ajoutés aux cellules, chacun à raison de 20 µg/ml. Le volume final est de 200 µl. Les plaques sont incubées 18 h à 37°C dans un incubateur humidifié à 5 % de gaz carbonique. Les plaques sont ensuite lavées avec du PBS contenant 0,05 % de Tween 20 et recouvertes avec un anticorps biotinylé anti-IFN-gamma (BD Pharmingen) à raison de 1 µg/ml. Après 2 h d'incubation à température ambiante, les plaques sont lavées en PBS Tween et traitées avec de la streptavidine conjuguée à de la peroxydase (Southern Biotechnology, Clinisciences). Les plaques sont incubées pendant une heure à température ambiante, puis lavées amplement. Après un dernier lavage, les cellules sécrétrices d'IFN-gamma sont visualisées en ajoutant une solution de 3-amino-9-ethylcarbazole. Les spots sont décomptés avec un analyseur d'image (Microvision Instruments) et les valeurs sont confirmées par comptage manuel. La fréquence des cellules productrices d'IFN-gamma est calculée en faisant la moyenne du nombre de spots sur les 3 exemplaires. La moyenne des spots dans les puits ne contenant pas d'antigène est soustrait. Les résultats sont présentés dans la Figure 3. Bien évidemment des contrôles positif (ConcanavalineA) et négatif (simple milieu RPMI sans cellule) sont aussi réalisés : On observe moins de 10 spots / million de cellules pour ce contrôle négatif et environ 500 spots / million de cellules pour le contrôle positif.

On constate que le F68 stimule légèrement la réponse cellulaire. Cet effet est surtout visible aux doses d'ADN de 1 et 10 µg. On n'observe plus d'effet à la dose de 50 µg d'ADN.

## Revendications

1. Une composition pharmaceutique comprenant un polynucléotide et au moins 2 % (poids / volume) d'un copolymère non-ionique de formule (I) OH(CH2CH2O)a(CH(CH3)CH2O)b(CH2CH2O)cH dans laquelle a, b and c sont tels que la portion polyoxypropylène possède un poids moléculaire compris entre 1450 and 2050 et les portions polyoxyethylène constituent entre 75 and 85 % (poids : poids) du copolymère.

2. Une composition selon la revendication 1, exempte de lipide cationique.

3. Une composition selon la revendication 1 ou 2, exempte de phosphate de sodium.

4. Une composition selon la revendication 3, comprenant un polynucléotide et 2 à 10 % (poids / volume) d'un copolymère non-ionique de formule (I).

5. Une composition selon l'une des revendications 1 à 4, comprenant un polynucléotide et environ 5 % (poids / volume) d'un copolymère non-ionique de formule (I).

6. Une composition selon l'une des revendications 1 à 5, qui comprend en outre un composé sélectionné dans le groupe constitué par le chlorure de sodium, le chlorure de potassium et le chlorure de magnésium, en quantité isotonique ou hypertonique.

7. Une composition selon l'une des revendications 1 à 6, dans laquelle le copolymère non-ionique est le F68.

8. Une composition selon l'une des revendications 1 à 7, dans laquelle le polynucléotide est un polynucléotide antisens.

9. Une composition selon l'une des revendications 1 à 7, dans laquelle le polynucléotide est capable de gouverner l'expression d'un polypeptide d'intérêt dans une cellule eucaryote.

10. Une composition selon la revendication 9, dans laquelle le polynucléotide est capable de gouverner l'expression dans une cellule eucaryote, d'un polypeptide spécifique d'un agent infectieux ou d'un état cancéreux.

11. Une composition selon la revendication 9, dans laquelle le polynucléotide est capable de gouverner l'expression d'un polypeptide capable de remédier à une déficience génétique.

12. Une composition selon l'une des revendications 8 à 11, dans laquelle le polynucléotide est de l'ADN.

13. L'usage d'un copolymère non-ionique de formule (I) OH(CH2CH2O)a(CH(CH3)CH2O)b(CH2CH2O)cH dans laquelle a, b et c sont tels que la portion polyoxypropylène possède un poids moléculaire compris entre 1450 et 2050 et les portions polyoxyethylène constituent entre 75 et 85 % (poids : poids) du copolymère dans la fabrication d'un médicament contenant un polynucléotide à titre de principe actif; le copolymère étant ajouté au polynucléotide à la concentration d'au moins 2 % (poids : volume) pour améliorer le transfert et/ou de l'expression du polynucléotide à l'intérieur des cellules de l'individu ayant besoin dudit médicament.

14. L'usage selon la revendication 13, dans lequel le copolymère est ajouté au nucléotide à la concentration de 2 à 10 %.

15. L'usage selon la revendication 14, dans lequel le copolymère est ajouté au nucléotide à la concentration d'environ 5 %.

16. L'usage selon l'une des revendications 13 à 15, dans lequel le médicament est exempt de lipide cationique ou de phosphate de sodium.

17. L'usage selon l'une des revendications 13 à 16, dans lequel le médicament comprend en outre un composé sélectionné dans le groupe constitué par le chlorure de sodium, le chlorure de potassium et le chlorure de magnésium, en quantité isotonique ou hypertonique.

18. L'usage selon l'une des revendications 13 à 17, dans lequel le copolymère y est defini selon la revendication 7.

19. L'usage selon l'une des revendications 13 à 18, dans lequel le polynucléotide y est défini selon l'une des revendications 8 à 12.

20. L'usage selon l'une des revendications 13 à 19, dans lequel le polynucléotide est capable de gouverner l'expression dans une cellule eucaryote d'un polynucléotide spécifique d'un agent infectieux ou d'un état cancéreux et dans lequel le médicament est destiné à induire une réponse immune à l'encontre de l'agent infectieux ou de l'état cancéreux.

21. L'usage selon la revendication 20, dans lequel la réponse immune est notamment de type humoral.

22. L'usage selon la revendication 20 ou 21, dans lequel la réponse immune protège contre une infection engendrée par l'agent infectieux ou contre un état cancéreux.

23. L'usage selon la revendication 20 ou 21, dans lequel la réponse immune a une action thérapeutique à l'encontre d'une infection engendrée par l'agent infectieux ou d'un état cancéreux.

24. Un procédé pour transférer un polynucléotide dans des cellules eucaryotes selon lequel on met en présence les cellules et le polynucléotide ; le polynucléotide étant formulé avec au moins 2 % (poids : volume) d'un copolymère non-ionique de formule (I) OH(CH2CH2O)a(CH(CH3)CH2O)b(CH2CH2O)cH dans laquelle a, b et c sont tels que la portion polyoxypropylène possède un poids moléculaire compris entre 1450 et 2050 et les portions polyoxyethylène constituent entre 75 et 85 % (poids : poids) du copolymère.

25. Un procédé selon la revendication 24, dans lequel le polynucléotide est formulé selon l'un des critères définis aux revendications 2 à 6.

26. Un procédé selon la revendication 24 ou 25, dans lequel le copolymère y est défini selon la revendication 7.

27. Un procédé selon la revendication 24, 25 ou 26, dans lequel le polynucléotide y est défini selon l'une des revendications 8 à 12.
